# FASCICULE DE BREVET EUROPEEN

(11) **EP 0 648 219 B1**
(45) Date de publication et mention de la délivrance du brevet: **26.08.1998**
(21) Numéro de dépôt: 93914778.1
(22) Date de dépôt: 29.06.1993
(51) Int. Cl.: C07H 13/06, C08B 37/00, C12N 15/74, C12N 1/21, C12P 19/26, A01N 63/02, A61K 31/70, A61K 31/715

(54) **SIGNAUX DE NODULATION DE RHIZOBIACEAE A LARGE SPECTRE D'HOTE**
BREITSPEKTRUM NODULATIONSIGNALE VON RHIZOBIACEEN
BROAD HOST SPECTRUM RHIZOBIACEAE NODULATION SIGNALS

(30) Priorité: 29.06.1992 FR 9207958
(43) Date de publication de la demande: 19.04.1995
(73) Titulaire: INSTITUT NATIONAL DE LA RECHERCHE AGRONOMIQUE (INRA), F-75007 Paris Cédex 07 (FR); CENTRE NATIONAL DE LA RECHERCHE SCIENTIFIQUE (CNRS), 75794 Paris Cédex 16 (FR)
(72) Inventeur: BROUGHTON, William, John, CH-1201 Genève (CH); DENARIE, Jean, Louis, Claude, F-31320 Castanet-Tolosan (FR); MAILLET, Fabienne, Chantal, Marie, F-31450 Pompertuzat (FR); PRICE, Neil Philip John, Complex Carbohydrate, Athens, GA 30602 (US); PROME, Danielle, Jeanne, Claudine, F-31320 Pechbusque (FR); PROME, Jean-Claude, Adrien, Paul, F-31320 Pechbusque (FR); RELIC, Biserka, CH-1227 Carouge (CH); TALMONT, Franck, Jean, Bernard, F-31500 Toulouse (FR)
(74) Mandataire: Ores, Irène
(86) Numéro de dépôt international: FR9300653
(87) Numéro de publication internationale: WO9400466

(56) Documents cités:
- EP-A- 0 330 715
- WO-A-91/15496
- CHEMICAL ABSTRACTS, vol. 110, 1989, Columbus, Ohio, US; abstract no. 232023c, K.SATO 'Preparation of tri-N-acylchitotrioses as Pharmaceuticals and Plant Growth Regulators.' page 689 ;colonne 1 ;

## Description

L'Invention est relative à l'obtention de nouveaux signaux de nodulation (facteurs Nod) à large spectre d'hôte.

Des bactéries du sol appartenant aux genres *Azorhizobium, Bradyrhizobium, Sinorhizobium* et *Rhizobium,* (désignées sous le terme général de *Rhizobia)* sont capables d'interagir avec les racines des légumineuses pour former des nodules dans lesquels elles fixent l'azote atmosphérique. Toutefois, seules certaines combinaisons bactéries/plantes conduisent à la nodulation, et la spécificité d'hôte des *Rhizobia* varie de façon importante [LONG, Cell. 56, 203 (1989)] ; [MARTINEZ et al., Crit. Rev. Plant Sci., 23, 483 (1990)] ; [DENARIE et al., in Molecular Signals in Plant-Microbe Communications, D.P.S. Verma Ed. p. 295-324 (CRC Press, Boca Raton, 1992)]. Certains *Rhizobia* (par exemple *R. leguminosarum* et *R. meliloti)* ne nodulent qu'un petit nombre d'espèces de légumineuses, alors qu'au contraire, d'autres ont un spectre d'hôte plus large et peuvent s'associer avec de nombreuses plantes.

La formation des nodules résulte de l'expression coordonnée de gènes de la plante et de gènes de la bactérie. L'expression des gènes de nodulation rhizobiaux (*nod*) est contrôlée par des gènes *nodD* de régulation, dont les produits sont activés par des flavonoides sécrétés par les racines des plantes. La capacité des protéines NodD à interagir avec les flavonoïdes de plantes de façon spécifique définit un premier niveau de spécificité d'hôte.

Il existe en outre deux catégories de gènes *nod* structuraux : les gènes communs et les gènes spécifiques. Les gènes *nodABC* sont communs à tous les *Rhizobia,* tandis que des gènes *nod* spécifiques d'espèce sont les déterminants majeurs de la spécificité d'hôte. Il a été montré que, à la fois, les gènes *nod* communs et les gènes *nod* spécifiques sont impliqués dans la production de facteurs Nod extracellulaires qui provoquent la déformation de poils racinaires chez les légumineuses. Certains des Inventeurs ont identifié chez *R. meliloti* des facteurs Nod, dénommés NodRm, qui sont des structures lipo-oligosaccharidiques, dont la biosynthèse est sous le contrôle des gènes communs *nodABC,* et qui sont des oligomères de glucosamines liées entre elles par des liaisons β-1,4, N-acylés sur la glucosamine terminale non réductrice et N-acétylés sur les autres résidus glucosamine (Demande PCT FR/9100283 aux noms de l'INSTITUT NATIONAL DE LA RECHERCHE AGRONOMIQUE et du CENTRE NATIONAL DE LA RECHERCHE SCIENTIFIQUE). La spécificité d'hôte est ensuite déterminée par la nature des substituants portés par ce squelette commun. Chez *R. meliloti,* la fonction des gènes majeurs de spécificité d'hôte *(nodH* et *nodPQ)* est de déterminer la sulfatation de ces facteurs lipo-oligosaccharidiques [ROCHE et al., Cell, 67, 1131 (1991)], tandis que chez *R. leguminosarum,* les gènes *nodFE* contrôlent la synthèse d'un résidu lipidique hautement insaturé [SPAINK et al., Nature, 354, 125, (1991)].

La souche de *Rhizobium* sp. NGR234 occupe une place unique chez les symbiotes des légumineuses ; elle possède en effet le spectre d'hôte le plus large de toutes les *Rhizobia* connues, et actuellement, on sait qu'elle provoque la nodulation de plus de 60 espèces de légumineuses. On compte en particulier parmi ces hôtes, la plupart des légumineuses présentant un important intérêt commercial, tels que par exemple le soja ou l'arachide. Le *Rhizobium* NGR234 peut en outre provoquer la nodulation de plantes n'appartenant pas aux légumineuses, telles que par exemple, *Parasponia andersonii.*

Les Inventeurs ont cherché à isoler et à identifier les facteurs Nod responsables du large spectre d'hôte de *Rhizobium* sp. NGR234, et sont parvenus à caractériser une nouvelle famille de facteurs Nod, dénommés facteurs NodNGR. Ces facteurs NodNGR sont des lipo-oligo-saccharides qui appartiennent à la même famille que les facteurs NodRm déja décrits par certains des Inventeurs (Demande PCT FR/9100283), mais présentent également des caractéristiques structurales qui permettent de les différencier des facteurs Nod Rm.
Premièrement, leur résidu glucosamine terminal réducteur est substitué en C6 par un autre sucre ;
Deuxièmement, leur glucosamine terminale non réductrice peut être estérifiée par un ou plusieurs groupes carbamyle ;
Troisièmement, l'atome d'azote qui est substitué par l'acide gras à longue chaîne est également méthylé.

En outre, les Inventeurs ont étudié la structure des facteurs Nod produits par diverses souches de Rhizobiacées, d'origine géographique très différente, et symbiotiques des hôtes les plus variés, comme *Rhizobium tropici* qui nodule les haricots et *Leucaena, Sinorhizobium fredii* qui nodule le soja, et *Azorhizobium caulinodans* symbiote de *Sesbania.* Ils ont constaté que les facteurs Nod produits par ces différentes souches possédaient au moins une des particularités structurales observées pour les facteurs NodNGR, telle que la présence d'un sucre sur la glucosamine terminale réductrice *(Sinorhizobium fredii, Azorhizobium caulinodans, Rhizobium phaseoli),* ou d'un groupement N-méthyle sur la glucosamine terminale non-réductrice *(Rhizobium tropici, Azorhizobium caulinodans).*

Les nouveaux facteurs Nod conformes à l'Invention qui présentent au moins une des trois caractéristiques structurelles mentionnées ci-dessus, seront dénommés ci-après, de manière générale, facteurs Nod de type NodNGR.

Les facteurs Nod de type NodNGR obtenus à partir de NGR234 seront plus particulièrement dénommés ci-après facteurs NodNGR. Des facteurs Nod obtenus à partir des autres souches étudiées par les Inventeurs, et possédant au moins l'une de ces caractéristiques sont représentatifs des facteurs Nod de type NodNGR. Il apparaît que l'origine des facteurs Nod de type NodNGR peut être très variée

La présente Invention a pour objet des facteurs Nod de formule générale (I) ci-après : 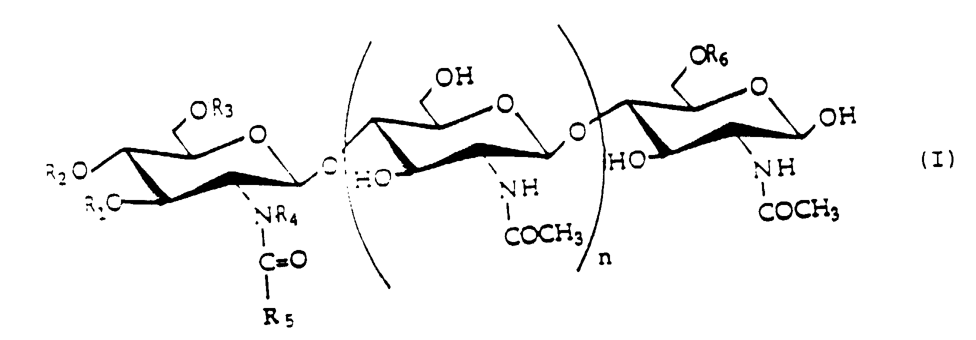 dans laquelle :
- R₁, R₂ et R₃ représentent un atome d'hydrogène, ou un groupe carbamyle ou un groupe acétyle ;
- R₅ représente la chaîne aliphatique d'un acide gras ;
- n est compris entre 1 et 4,
et caractérisés en ce que :
- un ou plusieurs des substituants R₁, R₂ ou R₃ est un groupe carbamyle, et/ou
- R₄ représente un groupe méthyle , et/ou
- R₆ représente un monosaccharide ou un oligo-saccharine, éventuellement substitués, liés à la glucosamine par liaison glycosidique .

Selon un mode de réalisation préféré de la présente Invention, R₅ représente la chaîne aliphatique d'un acide gras en C₁₀₋₂₄, de préférence C₁₄₋₂₀.

Selon une modalité préférée de ce mode de réalisation, R₅ représente la chaîne aliphatique de l'acide vaccénique ou de l'acide palmitique.

L'acide vaccénique et l'acide palmitique sont les substituants majeurs observés dans les préparations de facteurs NodNGR obtenus à partir de *Rhizobium* NGR234 ; toutefois, une grande variété d'acides gras, variant tant par le nombre d'atomes de carbone de la chaîne aliphatique, que par son degré d'insaturation, ou par la présence sur ladite chaîne aliphatique de substituants tels que des groupes hydroxyle, a été observée ; aucune influence de ces variations sur l'activité des facteurs NodNGR n'a été constatée par les Inventeurs.

Selon encore un autre mode de réalisation préféré de la présente Invention, R₆ est choisi parmi le groupe de monosaccharides comprenant le fucose et l'arabinose éventuellement substitués.

Selon une disposition avantageuse de ce mode de réalisation, R₆ répond à la formule générale (II) : 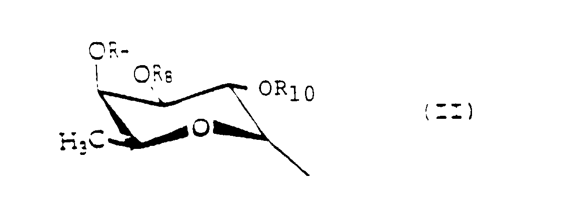 dans laquelle :
- R₇ et R₈ représentent un atome d'hydrogène, un groupe acétyle ou un groupe sulfate.
- R₁₀ représente un atome d'hydrogène ou un groupe méthyle

Selon une modalité préférée de cette disposition, R₇ est un atome d'hydrogène et R₈ un groupe sulfate.

Selon une autre modalité préférée de cette disposition, R₇ est un groupe acétyle et R₈ un atome d'hydrogène.

Selon encore une autre modalité préférée de cette disposition, R₇ et R₈ sont tous deux des atomes d'hydrogène.

Les facteurs NodNGR appartiennent à la même famille de molécules que les facteurs NodRm purifiés à partir de *R. meliloti:* tous possèdent le squelette commun de résidus de D-glucosamine liés entre eux par des liaisons β-1,4, N-acylé sur la glucosamine terminale non réductrice et N-acétylé sur les autres résidus. Ces similarités de structure recoupent les observations faites précédemment par certains des Inventeurs et confirment que chez tous les *Rhizobia,* la fonction des gènes *nodABC* est de contrôler la synthèse d'une ossature d'oligo-chitosane N-acylée et N-acétylée. Les travaux des Inventeurs mettent donc en évidence l'appartenance de facteurs Nod de toutes les *Rhizobiaceae* à la même famille chimique.

Dans les facteurs NodRm, la chaîne d'acide gras contient au moins une double liaison conjuguée qui semble jouer un rôle essentiel pour l'induction de méristèmes nodulaires ; au contraire, les facteurs NOdNGR sont N-acylés par de l'acide vaccénique ou de l'acide palmitique, et ne possèdent donc pas de double liaison conjuguée. Dans les facteurs NodNGR, l'atome d'azote qui est substitué par l'acide gras à longue chaîne est également méthylé. Ces observations permettent d'émettre l'hypothèse que certaines activités biologiques des lipo-oligosaccharides de type Nod exigeraient une configuration de structure définie à la jonction entre la partie lipidique et l'ossature saccharidique. Ces conditions de structure seraient assurées par la double liaison conjuguée dans un cas, et par le groupement N-méthyle dans l'autre cas .

La famille des facteurs NodNGR est très étendue. L'analyse par spectrométrie de masse utilisant l'ionisation par bombardement d'atomes rapides (FAB-MS) ainsi que l'analyse par résonance magnétique nucléaire ont montré l'existence de variations suivantes dans les substituants :
1) Le résidu de 2-O-méthyl-fucose peut être non substitué ou bien il peut être sulfaté en O-3 ou acétylé en O-4 ;
2) L'atome d'azote de la glucosamine terminale non réductrice peut être acylé par l'acide palmitique ou bien par l'acide vaccénique ;
3) Le nombre de substituants carbamyle sur la glucosamine terminale non réductrice varie entre aucun et deux.

Les combinaisons de ces différentes substitutions possibles conduisent à au moins 18 (= 3 x 2 x 3) structures possibles si les sites de substitutions carbamyles sont fixés, et leur nombre peut même être plus grand dans la mesure où le ou les sites de substitution par les groupes carbamyle peuvent varier entre les positions 0-3, 0-4, et 0-6. On peut raisonnablement supposer que c'est notamment cette diversité de structures qui est responsable du large spectre d'hôte des facteurs NodNGR.

L'Invention a également pour objet des souches de *Rhizobia* surproductrices de facteurs de type NodNGR, caractérisées en ce qu'elles contiennent au moins un plasmide recombinant exprimant un gène de régulation *nodD* de NGR234, et en particulier une souche de *Rhizobium* NGR234 surproductrice de facteurs Nod, obtenue en introduisant dans NGR234 un plasmide multicopie recombinant dénommé pA28, exprimant un gène de régulation *nodD* de NGR234, afin d'augmenter le nombre de copies de ce gène, ce qui a pour effet de multiplier par au moins 5 la quantité de facteurs Nod produite.

Le plasmide pA28 résulte de l'insertion d'un fragment *ECO*RI*-Pst*I du plasmide pNGRH6 [BASSAM et al. Mol. Plant-Microbe Interact, 1, 161,(1988)], portant la région *nodD1* de NGR234, dans le plasmide pRK7813 [JONES and GUTTERSON, Gène, 61, 299-306. (1987)].

L'Invention concerne également un procédé de production de facteurs NodNGR ou de type NodNGR, lequel procédé est caractérisé en ce qu'il comprend une étape au cours de laquelle on procède à la mise en culture d'au moins une souche de *Rhizobia* productrice desdits facteurs Nod.

De préférence, on choisira une souche dans laquelle un plasmide portant le gène de régulation *nodDl* de NGR234 a été introduit.

Selon un mode de mise en oeuvre préféré du procédé de production de facteurs de type NodNGR conforme à l'invention, il comprend en outre, une étape au cours de laquelle on extrait, à partir de ladite culture de souches de *Rhizobia,* une ou plusieurs fractions comprenant lesdits facteurs.

Selon une disposition préférée de ce mode de mise en oeuvre, les facteurs NodNGR sont extraits à partir du surnageant de culture, par chromatographie en phase inverse, par absorption sur une colonne de silice sur laquelle sont greffés des groupements hydrophobes, comme des résidus octadécyle, suivie d'une élution par du méthanol.

La présente Invention a également pour objet un agent de traitement de plantes comprenant en tant que constituant actif, au moins un facteur NodNGR ou de type NodNGR tel que défini plus haut, et qui est utilisable en particulier :
- comme un agent de stimulation des propriétés symbiotiques des légumineuses, notamment à l'égard de la fixation d'azote;
- comme un agent de stimulation des mécanismes de défense des plantes contre les pathogènes.

Préférentiellement, ledit agent de traitement des plantes comprend un mélange de facteurs NodNGR et/ou de facteurs de type NodNGR. Avantageusement, il peut également comprendre d'autres facteurs Nod, par exemple des facteurs de type NodRm.

Selon un mode de réalisation avantageux de l'agent de traitement de plantes conforme à la présente Invention, celui-ci est inclus dans un support solide, tel que des granulés, ou bien formulé sous la forme d'une composition d'enrobage de graines ou d'une solution ou suspension aqueuse pour pulvérisation, dans laquelle un ou des facteurs Nod conformes à l'Invention sont présents seuls ou associés à d'autres constituants, tels que par exemple d'autres facteurs Nod.

Selon un autre mode de réalisation avantageux de l'agent de traitement de plantes conforme à la présente Invention, un facteur ou chacun des constituants d'un mélange de facteurs Nod conformes à l'Invention sont présents dans les compositions d'enrobage ou les solutions ou suspensions aqueuses, à une concentration comprise entre 10⁻⁶ M et 10⁻¹⁴ M.

La présente Invention a en outre pour objet un agent thérapeutique, comprenant en tant que constituant actif au moins un facteur NodNGR ou de type NodNGR tel que défini plus haut.

Selon un mode de réalisation avantageux de cet agent thérapeutique, ledit facteur est présent dans l'agent thérapeutique à une concentration comprise entre 10⁻⁴ M et 10⁻⁸ M.

Outre les dispositions qui précèdent, l'Invention comprend encore d'autres dispositions qui ressortiront de la description qui va suivre.

Il doit être bien entendu, toutefois que ces exemples, ainsi que les dessins annexés, sont donnés uniquement à titre d'illustration de l'objet de l'Invention, dont ils ne constituent en aucune manière une limitation.

### EXEMPLE 1 : OBTENTION D'UNE SOUCHE DE BACTERIESRHIZOBIACEES SURPRODUCTRICE DE FACTEURS NodNGR

La manipulation de la souche NGR234 et de son ADN a été effectuée comme décrit par BROUGHTON et al. (Arch. Microbiol. 141, 14 (1985) et PERRET et al (Proc. Natl. Acad. Sci. 88, 1923 (1991), ou en utilisant des techniques classiques (J. SAMBROOK et al., Molecular Cloning. A Laboratory Manual, Cold Spring Harbor laboratory Press, Cold Spring Harbor (1989).

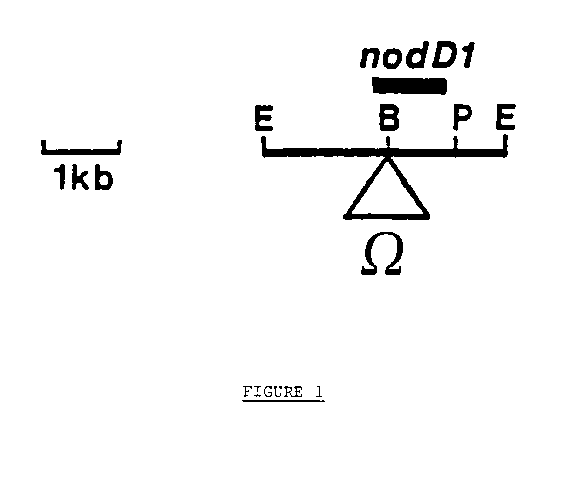

Un fragment *Eco*R1 de 2,9kb contenant la région *nodD1* de NRG234 a été excisé du plasmide pNGRH6 [BASSAM et al., Mol., Plant-Microbe Interact. 1, 161, (1988)] puis digéré par *Pstl.* Le fragment résultant *Eco*R1*-Ps*t1 de 2,2 kb a été cloné dans pRK7813 au site *Pstl* pour donner le plasmide pA28. pA28 est réintroduit dans *Rhizobium* sp.NGR234 (Rif^{R}) par conjugaison. La souche résultante NGR(pA28) est surproductrice de facteurs Nod. Elle est Nod⁺ sur *L. leucocephala, M. atropurpureum, et V. unguiculata.* La figure 1 représente la carte de restriction du fragment *Eco*R1*.* Les sites de restriction sont désignés comme suit :
B = *Bam*H1*,* E = *Eco*R1, P = *Pst1,*

### EXEMPLE 2 : PURIFICATION DES FACTEURS NodNGR

Les bactéries NGR234 ou NGR234 (pA28) sont mises en culture à 27 °C sur milieu B+D (W.J. BROUGHTON and M.J. DILWORTH, Biochem. J. 125, 1075 (1971) contenant 12 mM de succinate, 6 mM de glutamate, 1 ml/litre de solution de vitamine B5 GAMBORG (Sigma, St. Louis, Mo) (= RMM3), jusqu'à la fin de la phase logarithmique.

Pour l'analyse par chromatographie en couche mince, 30 ml de bactéries (NGR 234) en culture sont induites avec 10⁻⁶ M d'apigénine, qui est un inducteur de l'expression des gènes *nodD* de NGR234, en présence de 1 µCi/ml de sulfate de sodium marqué au soufre 35, ou d'acétate de sodium marqué au carbone 14.

Les surnageants sont extraits sur des cartouches SEPPAK C₁₈ (Waters Assoc., Milford, MA), lavés avec de l'eau distillée et élués au méthanol. Les extraits méthanoliques concentrés sont déposés sur des plaques de SILICAGEL 60 G (MERCK, DARMSTADT); la chromatographie est effectuée dans un mélange chloroforme:méthanol: 5 M NH₄OH (5:4:1 en volume), et la plaque déposée sur un film FUJI RX (Fuji Photo Film Co, TOKYO).

L'induction des gènes *nod* par 10⁻⁶M d'apigénine en présence d'acétate marqué au ¹⁴C ou de sulfate marqué au ³⁵S donne deux taches radioactives sur des plaques de CCM. L'extraction des principes actifs contenus dans l'une et l'autre de ces taches permet l'obtention de substances qui déforment les poils racinaires de *Macroptilium.* Ceci montre que NGR234 sécrète des facteurs Nod contenant du soufre. En revanche, aucune tache ne peut être détectée à partir d'un dérivé de NGR234 dans lequel les gènes *nodABC* ont été délétés. D'autre part, si l'on augmente le nombre de copies du gène *nodD1* (gène régulateur) en introduisant le plasmide pA28 dans NGR234, on observe un accroissement de production de facteurs Nod, la quantité produite étant multipliée par 5 à 10.

Pour l'analyse chimique, les facteurs Nod ont été isolés à partir du surnageant de cultures de NGR234 contenant le plasmide pA28, après induction par l'apigénine. Cette production à plus grande échelle est effectuée à partir de 50 litres de culture de bactéries NGR234(pA28) préalablement induites par l'apigénine dans du milieu RMM3. Le matériel lipophile est récupéré sur une colonne phase inverse C₁₈ (LICHROSORB-18, 40µ, MERCK, DARMSTADT). La colonne est lavée avec 50 fois son volume d'eau distillée et éluée par 10 volumes de méthanol. La solution méthanolique est évaporée sous vide et diluée par 100 ml d'eau distillée. Après filtration, la solution aqueuse est extraite par 50 ml d'acétate d'éthyle pour extraire en particulier l'apigénine. La solution aqueuse est concentrée et les composés hydrosolubles sont séparés par HPLC préparative sur une colonne C₁₈ en phase inverse. L'élution est suivie à 206 nm. Le solvant est un gradient d'acétonitrile dans l'eau.

Deux pics majeurs dénommés fractions A et B ont été collectés de la sorte. La fraction A (0,3 mg/l de la culture de départ) co-élue avec le matériel provenant de la culture marquée au soufre 35, alors que la fraction B (0,5 mg/l de la culture originelle) n'est pas marquée dans ces conditions. La comparaison des activités biologiques de ces deux fractions est décrite ci-dessous, à l'exemple 4.

### EXEMPLE 3 : CARACTERISATION DES FACTEURS NodNGR

L'hydrolyse par l'acide trifluoroacétique (4 M pendant 4 heures à 100 °C) de chacune des deux fractions libère des sucres et des acides gras. Les sucres ont été identifiés comme étant la D-glucosamine, la N-méthyl-D-glucosamine, et le 2-O-méthyl-L-fucose, que ce soit par chromatographie en phase vapeur, par spectrométrie de masse, (CPV-SM) de leur dérivés d'acétate d'alditol, ou bien par analyse en chromatographie en phase gazeuse de leur (+)-2-butanol-glycosides. Deux acides ont été identifiés : le composant le plus important comme étant l'acide vaccénique (acide 11-Z-octadécénoïque) pendant que le composant mineur (environ 20% du total) a été identifié comme étant l'acide palmitique. L'existence d'un squelette commun constitué d'oligomères pentamériques de N-acétyl-glucosamine, portant plusieurs substituants a été déduite du spectre de masse FAB qui fait apparaître des séries d'ions séparées par 203 unités de masse (masse moléculaire d'un résidu N-acétyl-glucosamine).

La fraction A est un mélange de plusieurs composés sulfatés, comme le confirme la facilité avec lequel le radical SO₃ est perdu en mode ion positif. Le poids moléculaire du composant majeur, déduit du spectre d'ions négatifs, est de 1595. D'autres composants dont la masse est inférieure de 43 ou de 26 unités de masse, ou une combinaison des deux, ont été détectés. Cette dernière différence correspond à celle qui existe entre le poids moléculaire de l'acide vaccénique et le poids moléculaire de l'acide palmitique. De même, la différence de 43 unités de masse, qui était répétée deux fois, a été attribuée à la présence ou l'absence de groupes CO-NH supplémentaires (résidus carbamyles). Le fait que cette empreinte de trois pics séparés par 43 unités de masse accompagnés de pics satellites distants de 26 unités de masse soit observée à chaque rupture de liaison glycosidique en mode d'ions positifs (formation d'ions oxénium) justifie la localisation des groupements carbamyles sur la glucosamine terminale non réductrice. De plus, si l'on soustrait de la masse des ions oxéniums à m/z 440, 483, et 526 la masse d'un résidu vaccényle (cétène) et, selon les cas, celle de zéro, un ou deux groupes carbamyles (43 unités de masse), on obtient la masse de l'ion oxénium d'une méthyl-glucosamine. Ceci permet de localiser la N-méthyl-glucosamine à l'extrémité non réductrice de l'oligosaccharide.

La fraction B est aussi un mélange. Deux composants majeurs ont été identifiés (poids moléculaires respectifs 1557 et 1515). La différence de 42 unités de masse entre ces deux composants suggère que le second est une forme monoacétylée du premier. D'autre part, comme dans la fraction A, d'autres composants dans lesquels la masse est plus faible de 43 ou 26 unités de masse sont également présents. Comme pour les composés de la fraction A, les groupes carbamyles sont localisés sur la N-méthyl-glucosamine terminale non réductrice portant le groupe N-acyle. Par contre le groupe acétyle additionnel n'étant présent dans aucun des ions oxéniums observés, celui-ci est localisé près de l'extrémité réductrice.

Le spectre de RMN du carbone 13 est compatible avec la présence de groupes carbamyles (δ = 161,09, 160,62 et 159,80 ppm) et celle des autres substituants décrits précédemment. Le spectre de RMN du proton attribue des configurations β pour les liaisons entre glucosamines et α pour la liaison entre le 2-O-méthyl fucose et la glucosamine réductrice. Le spectre COSY montre une corrélation entre H-5 du fucose et un proton déblindé à δ = 4,53 ppm pour les composés de la fraction B. Ceci permet d'attribuer la position du groupe acétyle comme étant situé en O-4 du 2-O-méthyl-fucose. Parallèlement, le spectre COSY des composés de la fraction A montre une corrélation entre H-2 du 2-O-méthyl-fucose (3,67 ppm) et le proton H-3 déblindé à δ = 4,65 ppm, permettant de localiser le groupe sulfate sur O-3 du 2-O-méthyl-fucose. Cette dernière attribution est confirmée par l'analyse des sucres obtenus par hydrolyse de la fraction A réduite et perméthylée, montrant la présence de diméthyl-2,4-fucose. De plus, l'identification de triméthyl 1,2,3,5 glucosaminitol dans les produits d'hydrolyse des fractions A ou B réduites et perméthylées permettent d'affirmer que le méthyl-fucose est glycosidiquement lié sur O-6 de la glucosamine réductrice. Enfin, cette analyse indique également des liaisons 1-4 entre les diverses glucosamines. Les conditions de méthylation produisant l'élimination simultanée des groupes esters (acétates et carbamates), la position de ces groupes ne peut être établie par cette méthode. Toutefois, les composants majeurs des fractions A et B sont bi-carbamylés, alors que les espèces dépourvues de substituants carbamyle sont en minorité.

### EXEMPLE 4 : TEST DE CROISSANCE DES POILS RACINAIRES (Hai) ET DE DEFORMATION DES POILS RACINAIRES (Had) PROVOQUES PAR LES FACTEURS NOD SULFATES ET NON SULFATES DE RHIZOBIUM NGR234 SUR MACROPTILIUM ATROPURPUREUM, MEDICAGO SATIVA, VICIA SATINA, ET VIGNA UNGUICULATA.

Les deux fractions A et B, (sulfatée et non sulfatée) ont été séparées par chromatographie HPLC en phase inverse selon le protocole décrit dans l'exemple 2, et ont été testées séparément pour leur activité biologique.

Les tests Had (déformation des poils racinaires) sur *M. sativa* et Hai (prolifération et recourbement des poils racinaires) sur V. *sativa* ont été effectués comme décrits par ROCHE et al. [Cell., 76, 1131 (1991)]. Dans les tests Had pour *Macroptilium* et *Vigna,* des plantules stériles sont placées dans des tubes Eppendorf modifiés (dans lesquels le bouchon et une partie du fond ont été enlevés), et les tubes Eppendorf contenant les plantules sont suspendus dans des tubes test dont le fond est peint en noir afin de protéger les racines de la lumière, de façon à ce que l'extrémité des racines soit en contact avec 10 ml de milieu B + D. Après une incubation de 60 heures ( 16 heures de jour, 30°C ; 8 heures de nuit 20°C), les racines sont enlevées, colorées au bleu de méthylène, et examinées sous microscope à inversion. Les systèmes racinaires montrant clairement des branchements ou des recourbements (ramifications prolifiques ou recourbements à plus d'un endroit du système racinaire) sont indiqués comme Had⁺. Les racines recouvertes de poils racinaires sont indiquées comme Hai⁺. 10 plantes ont été utilisées pour chaque traitement et dilution. En outre, 40 *(Macroptilium* et *Vigna)* et 60 *(Medicago* et *Vicia)* plantules sont utilisés comme plantes de contrôle (cultivées sur milieu seul), afin d'estimer la variabilité intrinsèque d'une plante à l'autre des caractères Had et Hai.

Les résultats sont représentés au tableau I ci-après. Ces résultats montrent le nombre de plantes (sur 10) qui montrent une activité Had ou Hai positive. Les nombres sont suivis par ^{S} quand la proportion de Had⁺ ou Hai⁺ est significativement plus élevée (probabilité P = 0,05) parmi les plantes traitées que parmi les contrôles (analyse faite en utilisant le test de Fisher).
- NodRm-IV(Ac,S) est le facteur Nod sulfaté majeur de la souche *R. meliloti* 2011. Il est utilisé comme contrôle positif de l'activité Had sur *Medicago.*
- NodRm-IV(Ac) est un facteur Nod non-sulfaté de mutants NodH⁻ de *R. meliloti.* Il est utilisé comme contrôle posifif de l'activité Hai sur *Vicia.*

**TABLEAU 1**

| CONCENTRATION FACTEUR Nod (M) | | | | | | | |
|---|---|---|---|---|---|---|---|
| | 10⁻⁷ | 10⁻⁸ | 10⁻⁹ | 10⁻¹⁰ | 10⁻¹¹ | 10⁻¹² | 10⁻¹³ |
| *Macroptilium* (Had) | | | | | | | |
| NodNRG sulfaté | nt | 10^{s} | 10^{s} | 7^{s} | 3^{s} | 0 | |
| NodNGR non-sulfaté | nt | 10^{s} | 9^{s} | 4^{s} | 1 | 0 | |

| *Vigna* (Had) | | | | | | | |
|---|---|---|---|---|---|---|---|
| NodNRG sulfaté | 10^{s} | 10^{s} | 10^{s} | 10^{s} | 10^{s} | 9^{s} | 6^{s} |
| NodNGR non-sulfaté | 10^{s} | 10^{s} | 10^{s} | 8^{s} | 5^{s} | 4 | nt |

| *Medicago* (Had) | | | | | | | |
|---|---|---|---|---|---|---|---|
| NodNRG sulfaté | 9^{s} | 8^{s} | 9^{s} | 5^{s} | 3^{s} | 1 | |
| NodNGR non-sulfaté | 5^{s} | 1 | 2 | 0 | 1 | 1 | |
| NodRM-IV(AC,S) | nt | 9^{s} | 8^{s} | 7^{s} | 6^{s} | 4^{s} | |

| *Vicia* (Hai) | | | | | | | |
|---|---|---|---|---|---|---|---|
| NodNRG sulfaté | 4^{s} | 5^{s} | 3^{s} | 0 | 0 | 0 | |
| NodNGR non-sulfaté | 10^{s} | 10^{s} | 9^{s} | 2^{s} | 0 | 0 | |
| NodRM-IV(Ac) | nt | 10^{s} | 10^{s} | 8^{s} | 2^{s} | 0 | |
| nt = non testé. | | | | | | | |

Dans les tests de déformation des poils racinaires (Had) effectués sur des plantes hôtes de NGR234, les deux groupes de facteurs NodNGR sont actifs à des concentrations aussi faibles que 10⁻¹⁰M/10⁻¹¹M sur *Macroptilium* et 10⁻¹¹M/10⁻¹²M sur *Vigna.* En outre, sur *Vigna,* les facteurs NodRm induisent non seulement des déformations du chevelu racinaire, mais également l'apparition de nombreux poils racinaires, ainsi que leur recourbement (Hai).

Les facteurs NodRm sulfatés obtenus à partir de *R. meliloti* sont Had⁺ sur *Medicago sativa,* et Hai⁻ sur *Vicia sativa supsp. nigra.* Au contraire, les facteurs NodRm non sulfatés sécrétés par des mutants NodH⁻ de R. *meliloti* sont Had⁻ sur *Medicago* et Hai⁺ sur *Vicia.* De manière intéressante, les facteurs NodNGR sulfatés et non sulfatés, présentent une activité biologique sur ces deux légumineuses. Les facteurs NodNGR sulfatés sont 10000 fois plus actifs que les facteurs non sulfatés sur *Medicago,* et provoquent la déformation du chevelu racinaire à des concentrations inférieures à 10⁻¹¹ molaire. Les facteurs NodNGR sulfatés diffèrent des facteurs NodRm sulfatés par de nombreux critères : par exemple, la présence de groupes carbamyle et d'un résidu méthyl-fucose, la localisation du groupe sulfate sur le fucose au lieu de la glucosamine, l'absence de double liaison conjuguée sur la chaîne acyle substituant l'azote, et la présence d'un groupe méthyle substituant l'azote. Cependant, les deux types de facteurs sont actifs sur *Medicago,* et leur activité diminue d'environ 10.000 fois quand le groupe sulfate est enlevé, ce qui démontre que *Medicago* est très sensible aux facteurs *Nod* sulfatés. Au contraire, chez *Vicia,* les composés non sulfatés sont plus actifs, et la déformation du chevelu racinaire esc observée à des concentrations inférieures à 10⁻¹¹M.

## Revendications

1. Facteur Nod, dénommé facteur de type NodNGR de formule générale (I) ci-après : 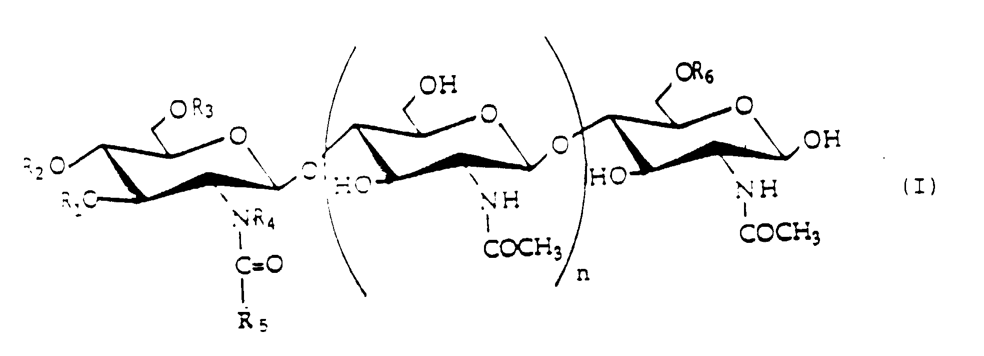 dans laquelle :
- R₁, R₂ et R₃ représentent un atome d'hydrogène, ou un groupe carbamyle ou un groupe acétyle ;
- R₅ représente la chaîne aliphatique d'un acide gras ;
- n est compris entre 1 et 4,
et caractérisé en ce que :
- un ou plusieurs des substituants R₁, R₂ ou R₃ est un groupe carbamyle, et/ou
- R₄ représente un groupe méthyle , et/ou
- R₆ représente un monosaccharide ou un oligo-saccharide, éventuellement substitués, liés à la glucosamine par liaison glycosidique .

2. Facteur Nod selon la Revendication 1, caractérisé en ce que R₅ représente la chaîne aliphatique d'un acide gras en C₁₀₋₂₄, de préférence C₁₄₋₂₀.

3. Facteur Nod selon la Revendication 2, caractérisé en ce que R₅ représente la chaîne aliphatique de l'acide vaccénique ou de l'acide palmitique.

4. Facteur Nod selon l'une quelconque des Revendications 1 à 3, caractérisé en ce que R₆ est choisi parmi le groupe de monosaccharides comprenant le fucose et l'arabinose, éventuellement substicués.

5. Facteur Nod selon la Revendication 4, caractérisé en ce que R₆ répond à la formule générale (II) : 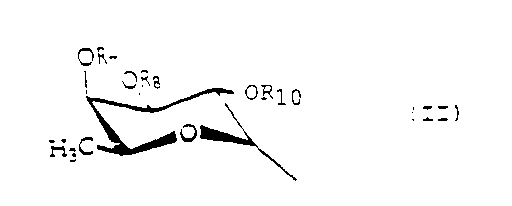 dans laquelle :
- R₇ et R₈ représentent un atome d'hydrogène, un groupe acétyle ou un groupe sulfate.
- R₁₀ représente un atome d'hydrogène ou un groupe méthyle.

6. Facteur Nod selon la Revendication 5, caractérisé en ce que R₇ est un atome d'hydrogène et R₈ un groupe sulfate.

7. Facteur Nod selon la Revendication 5, caractérisé en ce que R₇ est un groupe acétyle et R₈ un atome d'hydrogène.

8. Facteur Nod selon la Revendication 5, caractérisé en ce que R₇ et R₈ sont tous deux des atomes d'hydrogène.

9. Procédé de production de facteurs de type NOdNGR selon l'une quelconque des Revendications 1 à 8, lequel procédé est caractérisé en ce qu'il comprend une étape au cours de laquelle on procède à la culture d'au moins une souche de *Rhizobiacée* productrice desdits facteurs Nod, et une étape au cours de laquelle on procède à la purification desdits facteurs NodNGR à partir de la culture.

10. Procédé selon la Revendication 9, caractérisé en ce que ladite souche est une souche surproductrice de facteurs NodNGR, comprenant au moins un plasmide portant un ou plusieurs gènes de régulation *nodD* de Rhizobiacées choisies dans le groupe comprenant les genres *Rhizobium, Azorhizobium, Bradyrhizobium, Sinorhizobium.*

11. Procédé selon la Revendication 10, caractérisé en ce que ledit plasmide résulte de l'insertion d'un fragment *Eco*RI*-Pst*I comprenant le gène de régulation *nodD1* de la souche NGR234 dans le plasmide pRK7813.

12. Procédé selon une quelconque des revendications 9 ou 11, caractérisé en ce qu'il comprend en outre, une étape au cours de laquelle on extrait, à partir de ladite culture de Rhizobiacées, une ou plusieurs fractions comprenant les facteurs de type NodNGR.

13. Procédé selon la Revendication 12, caractérisé en ce que les facteurs de type NodNGR sont extraits à partir du surnageant de culture, par absorption sur une colonne de silice sur laquelle sont greffés des groupements hydrophobes, suivie d'une élution par du méthanol.

14. Souches de *Rhizobiacées* surproductrices de facteurs de type NodNGR, pour la mise en oeuvre du procédé selon la Revendication 10, caractérisées en ce qu'elles contiennent au moins un plasmide recombinant portant un ou plusieurs gènes de régulation *nodD* de Rhizobiacées choisies dans le groupe comprenant les genres *Rhizobium, Azorhizobium, Bradyrhizobium, Sinorhizobium.*

15. Agent de traitement de plantes caractérisé en ce qu'il comprend en tant que constituant actif, au moins un facteur de type NodNGR ou un mélange de facteurs de type NodNGR, selon l'une quelconque des Revendications 1 à 8.

16. Agent de traitement des plantes, selon la Revendication 15, caractérisé en ce qu'il comprend en outre d'autres facteurs Nod.

17. Agent de traitement de plantes selon la Revendication 16, caractérisé en ce qu'il est inclus dans un support solide, ou formulé sous la forme d'une composition d'enrobage de graines ou d'une solution ou suspension aqueuse pour pulvérisation.

18. Agent de traitement de plantes selon la Revendication 16, caractérisé en ce que le facteur de type NodNGR ou chacun des constituants du mélange de facteurs de type NodNGR est présent à une concentration comprise entre 10⁻⁶ M et 10⁻¹⁴ M.

## Patentansprüche

1. Nod-Faktor, insbesondere ein Faktor vom Typ NodNGR gemäß folgender allgemeiner Formel (I): 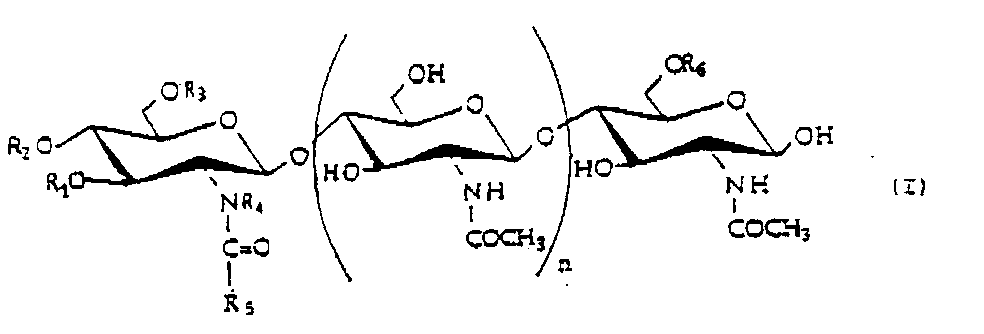 worin:
- R₁, R₂ und R₃ ein Wasserstoffatom oder eine Carbamylgruppe oder eine Acetylgruppe darstellen;
- R₅ eine aliphatische Kette einer Fettsäure darstellt;
- n zwischen 1 und 4 liegt,
dadurch gekennzeichnet, daß:
- eine oder mehrere der Substituenten R₁, R₂ oder R₃ eine Carbamylgruppe ist und/oder
- R₄ eine Methylgruppe darstellt und/oder
- R₆ ein Monosaccharid oder ein Oligosaccharid darstellt, ggf. substituiert, über eine glycosidische Bindung an das Glucosamin gebunden.

2. Nod-Faktor nach Anspruch 1, dadurch gekennzeichnet, daß R₅ die aliphatische Kette einer C₁₀₋₂₄ Fettsäure, vorzugsweise C₁₄-₂₀, darstellt.

3. Nod-Faktor nach Anspruch 2, dadurch gekennzeichnet, daß R₅ die aliphatische Kette der Vaccensäure oder der Palmitinsäure repräsentiert.

4. Nod-Faktor nach irgendeinem der Ansprüche 1 bis 3, dadurch gekennzeichnet, daß R₆ ausgewählt ist aus der Gruppe der Monosaccharide, die Fucose und Arabinose, ggf. substituiert, umfaßt.

5. Nod-Faktor nach Anspruch 4, dadurch gekennzeichnet, daß R₆ der allgemeinen Formel (II) entspricht: 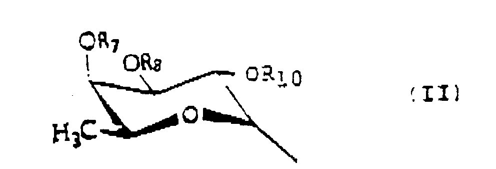 worin:
- R₇ und R₈ ein Wasserstoffatom, eine Acetylgruppe oder eine Sulfatgruppe darstellen;
- R₁₀ ein Wasserstoffatom oder eine Methylgruppe darstellt.

6. Nod-Faktor nach Anspruch 5, dadurch gekennzeichnet, daß R₇ ein Wasserstoffatom und R₈ eine Sulfatgruppe ist.

7. Nod-Faktor nach Anspruch 5, dadurch gekennzeichnet, daß R₇ eine Acetylgruppe und R₈ ein Wasserstoffatom ist.

8. Nod-Faktor nach Anspruch 5, dadurch gekennzeichnet, daß R₇ und R₈ beide Wasserstoffatome sind.

9. Verfahren zur Herstellung eines Faktors vom Typ NodNGR gemäß irgendeinem der Ansprüche 1 bis 8, wobei das Verfahren dadurch gekennzeichnet ist, daß es eine Stufe umfaßt, im Verlauf derer man wenigstens eine Kultur eines Stammes von Rhizobiaceae ansetzt, die die Nod-Faktoren produzieren und eine Stufe, im Verlauf derer man die Reinigung der NodNGR-Faktoren aus der Kultur durchführt.

10. Verfahren nach Anspruch 9, dadurch gekennzeichnet, daß der Stamm ein überproduktiver Stamm an NodNGR-Faktoren ist, welcher wenigstens ein Plasmid umfaßt, das ein oder mehrere Regulationsgene nodD der Rhizobiaceae trägt, ausgewählt aus der Gruppe umfassend die Genera Rhizobium, Azorhizobium, Bradyrhizobium, Sinorhizobium.

11. Verfahren nach Anspruch 10, dadurch gekennzeichnet, daß das Plasmid sich ergibt aus der Insertion eines Fragmentes EcoRI-PstI, umfassend die Genregulation nodDl des Stammes NGR234 in das Plasmid pRK7813.

12. Verfahren nach irgendeinem der Ansprüche 9 oder 11, dadurch gekennzeichnet, daß es eine weitere Stufe umfaßt, im Verlauf derer man aus der Rhizobiaceae-Kultur eine oder mehrere Fraktionen, welche die Faktoren vom NodNGR-Typ umfassen, extrahiert.

13. Verfahren nach Anspruch 12, dadurch gekennzeichnet, daß die Faktoren vom Typ NodNGR aus dem Kulturüberstand durch Absorption auf einer Kieselgelsäure, auf welcher hydrophobe Gruppen aufgepfropft sind, extrahiert werden, gefolgt von einer Methanolelution.

14. Rhizobiaceae-Stämme, überproduktiv an Faktoren vom Typ NodNGR zur Durchführung des Verfahrens nach Anspruch 10, dadurch gekennzeichnet, daß sie wenigstens ein rekombinantes Plasmid enthalten, welches ein oder mehrere Regulationsgene nodD der Rhizobiaceae trägt, ausgewählt aus der Gruppe umfassend die Genera Rhizobium, Azorhizobium, Bradyrhizobium, Sinorhizobium.

15. Mittel zur Behandlung von Pflanzen, dadurch gekennzeichnet, daß es als aktiven Bestandteil wenigstens einen Faktor vom Typ NodNGR oder eine Mischung von Faktoren des Typs NodNGR, nach irgendeinem der Ansprüche 1 bis 8, enthält.

16. Mittel zur Behandlung von Pflanzen nach Anspruch 15, dadurch gekennzeichnet, daß es ferner andere Nod-Faktoren enthält.

17. Mittel zur Behandlung von Pflanzen nach Anspruch 16, dadurch gekennzeichnet, daß es in einem festen Träger eingeschlossen ist oder formuliert ist in Form einer Zusammensetzung von Samenumhüllungen oder einer Lösung oder einer wäßrigen Suspension zur Verstäubung.

18. Mittel zur Behandlung von Pflanzen nach Anspruch 16, dadurch gekennzeichnet, daß der Faktor vom Typ NodNGR oder jeder Bestandteil der Mischung der Faktoren vom Typ NodNGR in einer Konzentration zwischen 10⁻⁶ M und 10⁻¹⁴ M vorliegt,

## Claims

1. Nod factor, termed NodNGR-type factor, of the general formula (I) hereinbelow: 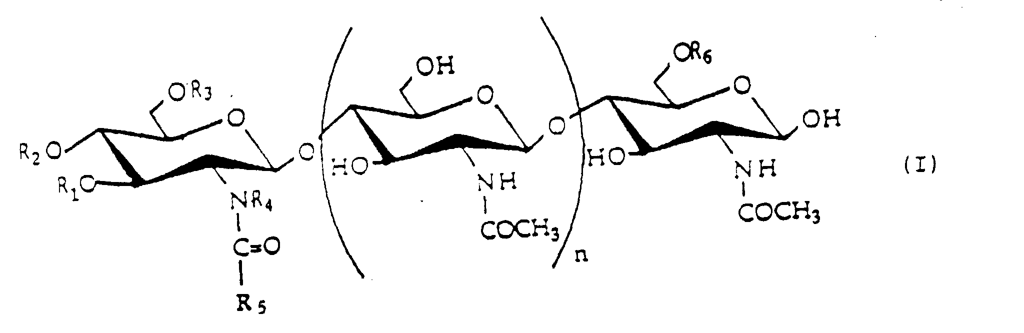 in which:
- R₁, R₂ and R₃ represent a hydrogen atom, a carbamoyl group or an acetyl group;
- R₅ represents the aliphatic chain of a fatty acid;
- n is between 1 and 4,
and wherein:
- one or more of the substituents R₁, R₂ or R₃ is a carbamoyl group, and/or
- R₄ represents a methyl group, and/or
- R₆ represents a monosaccharide or an oligosaccharide, optionally substituted, and linked to the glucosamine by a glycosidic linkage.

2. Nod factor as claimed in claim 1, wherein R₅ represents the aliphatic chain of a C₁₀₋₂₄, preferably a C₁₄₋₂₀, fatty acid.

3. Nod factor as claimed in claim 2, wherein R₅ is the aliphatic chain of vaccenic acid or palmitic acid.

4. Nod factor as claimed in any of claims 1 to 3, wherein R₆ is selected from amongst the monosaccharide group comprising optionally substitued fucose and optionally substituted arabinose.

5. Nod factor as claimed in claim 4, wherein R₆ has the general formula (II) 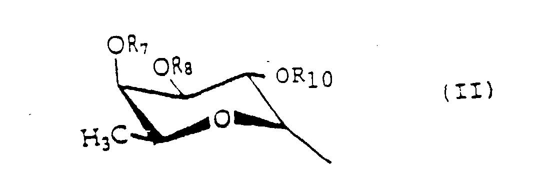 in which:
- R₇ and R₈ represent a hydrogen atom, an acetyl group or a sulfate group,
- R₁₀ represents a hydrogen atom or a methyl group.

6. Nod factor as claimed in claim 5, wherein R₇ is a hydrogen atom and R₈ a sulfate group.

7. Nod factor as claimed in claim 5, wherein R₇ is an acetyl group and R₈ a hydrogen atom.

8. Nod factor as claimed in claim 5, wherein both R₇ and R₈ are hydrogen atoms.

9. Process for the preparation of NodNGR-type factors as claimed in any of claims 1 to 8, which process comprises a step in which at least one strain of *Rhizobiaceae* producing said Nod factors is cultured, and a step in which said NodNGR factors are purified from the culture.

10. Process as claimed in claim 9, wherein this strain is a strain which overproduces NodNGR factors and comprises at least one plasmid having one or more regulator genes *nodD* from *Rhizobiaceae* selected from the group consisting of the genera *Rhizobium, Azorhizobium, Bradyrhizobium* and *Sinorhizobium.*

11. Process as claimed in claim 10, wherein the plasmid results from inserting an *Eco*RI*-Pst*I fragment comprising the regulator gene *nodD1* from strain NGR234 into plasmid pRK7813.

12. Process as claimed in either of claims 9 or 11, which comprises, moreover, a step in which one or more fractions comprising the NodNGR-type factors are extracted from said culture of rhizobiaceae.

13. Process as claimed in claim 12, wherein the NodNGR-type factors are extracted from culture supernatant by absorption onto a silica column to which hydrophobic groups are grafted, followed by elution with methanol.

14. Strains of *Rhizobiaceae* which overproduce NodNGR-type factors for carrying out the process as claimed in claim 10, which comprise at least one recombinant plasmid carrying one or more regulator genes *nodD* from *Rhizobiaceae* selected from the group consisting of the genera *Rhizobium, Azorhizobium, Bradyrhizobium* and *Sinorhizobium.*

15. Plant treatment agent, which comprises, as active ingredient, at least one NodNGR-type factor or a mixture of NodNGR-type factors as claimed in any of claims 1 to 8.

16. Plant treatment agent as claimed in claim 15, which comprises, additionally, other Nod factors

17. Plant treatment agent as claimed in claim 16, which is included in a solid carrier or formulated in the form of a coating composition for seed or an aqueous solution or suspension for spraying.

18. Plant treatment agent as claimed in claim 16, wherein the NODNGR-type factor or each of the ingredients of the mixture of NodNGR-type factors is present at a concentration of between 10⁻⁶M and 10⁻¹⁴M.
